# EUROPÄISCHE PATENTSCHRIFT

(11) **EP 4 048 395 B1**
(45) Veröffentlichungstag und Bekanntmachung des Hinweises auf die Patenterteilung: **02.07.2025**
(21) Anmeldenummer: 20796732.4
(22) Anmeldetag: 19.10.2020
(51) Int. Cl.: A61N 1/44, H05H 1/24

(54) **VORRICHTUNG ZUM AUSBILDEN VON PHYSIKALISCHEM PLASMA AN EINER OBERFLÄCHE EINES OBJEKTS**
DEVICE FOR FORMING PHYSICAL PLASMA ON A SURFACE OF AN OBJECT
DISPOSITIF POUR FORMER UN PLASMA PHYSIQUE SUR UNE SURFACE D'UN OBJET

(30) Priorität: 22.10.2019 DE 102019128538
(43) Veröffentlichungstag der Anmeldung: 31.08.2022
(73) Patentinhaber: Hochschule für Angewandte Wissenschaft und Kunst Hildesheim/Holzminden/Göttingen, 31134 Hildesheim (DE)
(72) Erfinder: VIÖL, Wolfgang, 37139 Adelebsen (DE); BORCHARDT, Thomas, 37115 Duderstadt (DE); BERTRAM, Joachim, 37127 Niemetal (DE)
(74) Vertreter: REHBERG HÜPPE + PARTNER
(86) Internationale Anmeldenummer: PCT/EP2020/079316
(87) Internationale Veröffentlichungsnummer: WO 2021/078667

(56) Entgegenhaltungen:
- EP-A1- 2 308 552
- EP-B1- 2 308 552
- WO-A1-2018/190499
- WO-A1-2019/107714

## Beschreibung

### TECHNISCHES GEBIET DER ERFINDUNG

Die Erfindung bezieht sich auf eine Vorrichtung zum Ausbilden von physikalischem Plasma an einer Oberfläche eines Objekts. Spezieller bezieht sich die Erfindung auf eine Vorrichtung zum Ausbilden des physikalischen Plasmas durch dielektrisch behinderte Entladungen gegenüber der Oberfläche des Objekts, die die Merkmalen des Oberbegriffs des unabhängigen Patentanspruchs 1 aufweist. Bei der Oberfläche des Objekts kann es sich insbesondere um eine Kopfhaut eines Menschen handeln.

### STAND DER TECHNIK

Eine Vorrichtung zur Erzeugung dielektrisch behinderter elektrischer Entladungen, die einen Hochspannungsanschluss und mehrere an den einen Hochspannungsanschluss kapazitiv angekoppelte, freiliegende Elektrodenoberflächen aufweisende und parallel zueinander zu distalen Enden hin langgestreckte Elektrodenkörper aufweist, ist aus der DE 10 2011 050 631 A1 bekannt. Hier sind die mehreren Elektrodenkörper aus Metall an den Hochspannungsanschluss angekoppelt, indem sie einem an dem Hochspannungsanschluss angeschlossenen Hochspannungsbus unter Zwischenordnung eines dielektrischen Festkörpers gegenüberliegen. Konkret enden dazu die mehreren Elektrodenkörper proximal in dem dielektrischen Festkörper, und der Hochspannungsbus weist ein die proximalen Enden der Elektrodenkörper in dem dielektrischen Festkörper U-förmig umschließendes Metallblech auf. Die Elektrodenkörper liegen mit ihren Haupterstreckungsrichtungen in einer Ebene, wobei ihre distalen Enden auf einer Linie mit stetigen Verlauf angeordnet sind. Mit der bekannten Vorrichtung können gegenüber einem geerdeten Objekt elektrische Entladungen hervorgerufen werden, die von den distalen Enden der Elektrodenkörper ausgehen. An oder nahe einer menschlichen Kopfhaut werden die elektrischen Entladungen zum Abtöten von Parasiten, wie beispielsweise Haarläusen und deren Vorformen, d. h. Nissen und Larven, hervorgerufen.

Aus der WO 2018/004300 A1 ist eine Vorrichtung zum Ausbilden von physikalischem Plasma durch dielektrisch behinderte Entladungen gegenüber einer Oberfläche eines Objekts bekannt, bei der einzelne, konkret drei mit Dielektrikum abgeschirmte verformbare Elektrodenkörper vorgesehen sind, um sie mit der Oberfläche des zu behandelnden Objekts in Kontakt zu bringen. Die Elektrodenkörper sind innerhalb einer ringförmigen Gegenelektrode angeordnet. Die bekannte Vorrichtung ist zur Behandlung von Haarausfall vorgesehen und wird dazu mit der gekrümmten Kopfhaut des Patienten in Kontakt gebracht. Das durch die dielektrisch behinderten Entladungen hervorgerufene Plasma entsteht dabei in den Bereichen kleiner Abstände zwischen der Kopfhaut und den dielektrischen Abschirmungen der an die Kopfhaut angedrückten Elektrodenkörper. Bei dieser bekannten Vorrichtung sind die einzelnen Elektrodenkörper mit ihren dielektrischen Abschirmungen von komplexem Aufbau, und die Vorrichtung lässt nur eine sehr lokale Erzeugung von Plasma und entsprechend eine nur sehr lokale Behandlung mit dem Plasma zu.

Aus der US 2016/0354614 A1 ist eine Vorrichtung zur Behandlung von Haarausfall bekannt, die mit Hilfe eines Helms eine Kammer über der Kopfhaut des jeweiligen Patienten ausbildet. In diese Kammer wird durch dielektrisch behinderte elektrische Entladungen erzeugtes kaltes Plasma eingeleitet. Diese bekannte Vorrichtung weist eine sehr aufwändige Gesamtkonstruktion auf.

Aus der WO 2017/162505 A1 ist eine Vorrichtung zur Behandlung von Haut mit kaltem Plasma bekannt. Die Vorrichtung umfasst ein Gehäuse mit einer Endfläche, einen Generator zum Erzeugen von kaltem Plasma, das reaktive Spezies zur Behandlung der Haut bereitstellt, und einen Manipulator zum Manipulieren der Haut, um die Exposition von Bakterien auf der Haut gegenüber den reaktiven Spezies während des Gebrauchs der Vorrichtung zu erhöhen. Der Generator ist während des Gebrauchs im Wesentlichen immer gleich von der Haut beabstandet. Der Manipulator erstreckt sich während des Gebrauchs zwischen dem Generator und der Haut und umfasst ein bewegliches Element, das zum Kontaktieren der Haut während des Gebrauchs der Vorrichtung ausgelegt ist. Der Manipulator kann eine Vielzahl von Vorsprüngen umfassen, die zum Kontaktieren der Haut während des Gebrauchs der Vorrichtung ausgelegt sind. Der Generator und der Manipulator können einstückig ausgebildet sein, wobei der Manipulator auf einer Oberfläche des Generators angeordnet ist.

Aus der DE 10 2015 112 200 A1 ist eine Elektrodenanordnung für eine Oberflächenbehandlung eines Körpers mit einem physikalischen Plasma bekannt. Die Elektrodenanordnung weist einen Hochspannungsanschluss zum Anschließen an einen Ausgang einer Wechselhochspannungsquelle, mehrere an den Hochspannungsanschluss angeschlossene linienförmige Elektrodenkörper, die mit einem Dielektrikum ummantelt und einzeln kapazitiv an den Hochspannungsanschluss gekoppelt sind, und einen an die Elektrodenkörper angrenzenden Behandlungsraum auf, in den der Körper für die Plasmabehandlung einzubringen ist. Die Elektrodenkörper umfassen jeweils einen bogenförmigen Bereich, in dem der jeweilige linienförmige Elektrodenkörper seine Richtung um mindestens 90° ändert. Der Behandlungsraum befindet sich zumindest teilweise zwischen den bogenförmigen Bereichen der Elektrodenkörper.

Aus der nachveröffentlichten DE 10 2018 126 492 A1 ist ein Plasma-Behandlungsgerät bekannt, das zur Behandlung einer Oberfläche mit einem dielektrisch behinderten Plasma ausgebildet ist. Das Plasma-Behandlungsgerät weist einen Grundkörper, der mindestens eine flächige und der zu behandelnden Oberfläche zugewandten Behandlungsseite aufweist, eine mindestens eine Elektrode umfassende Elektrodenanordnung und ein Dielektrikum auf, das die mindestens eine Elektrode zu der zu behandelnden Oberfläche hin vollständig abdeckt. Die Elektrode ist über eine Hochspannungszuleitung mit einem Hochspannungssignal beaufschlagbar. Eine an der Behandlungsseite des Grundkörpers angeordnete Noppenanordnung, die eine Vielzahl von Noppen aufweist, ermöglicht die Kombination einer effektiven Plasmabehandlung mit einer effektiven mechanischen Behandlung dadurch, dass sich die mindestens eine Elektrode der Elektrodenanordnung in mindestens eine Noppe der Noppenanordnung hinein erstreckt.

Aus der ebenfalls nachveröffentlichten DE 10 2018 126 489 A1 ist ein weiteres Plasma-Behandlungsgerät zur Behandlung einer Oberfläche mit einem dielektrisch behinderten Plasma bekannt, das eine mindestens eine Elektrode aufweisende Elektrodenanordnung, ein Dielektrikum, das die Elektrode zu der zu behandelnden Oberfläche hin vollständig abgedeckt, und ein Gehäuse aufweist. Die Elektrode ist über eine Hochspannungszuleitung mit einem Hochspannungssignal beaufschlagbar. Die Kombination einer effektiven Plasmabehandlung mit einer effektiven mechanischen Behandlung der zu behandelnden Oberfläche wird hier dadurch ermöglicht, dass das Plasma-Behandlungsgerät einen Bürstenkopf umfasst, der ein Borstenfeld und einen Borstenträger mit einer Basisfläche aufweist. Das Borstenfeld weist eine Vielzahl flexibler Borsten und Zwischenräume zwischen den Borsten auf. Die Borsten ragen von der Basisfläche des Borstenträgers in Richtung einer Auflagefläche ab, welche durch die von der Basisfläche abgewandten Enden der längsten Borsten des Borstenfelds definiert wird.

Eine Vorrichtung zum Ausbilden von physikalischem Plasma durch dielektrisch behinderte Entladungen zur Hautbehandlung, die die Merkmale des Oberbegriffs des unabhängigen Patentanspruchs 1 aufweist, ist aus der WO 2018/190499 A1 bekannt. Die Abstandhalter dieser Vorrichtung umfassen Röhren aus Dielektrikum, die jeden einzelnen Elektrodenkörper mit radialem Abstand umgeben, und mit axial über diesen überstehen. Um das Erzeugen des physikalischen Plasmas zu erleichtern sind die Elektrodenkörper mit spitzen Enden versehen.

### AUFGABE DER ERFINDUNG

Der Erfindung liegt die Aufgabe zugrunde, eine Vorrichtung zum Ausbilden von physikalischem Plasma durch dielektrisch behinderte Entladungen aufzuzeigen, die einen einfachen Aufbau aufweist und dennoch gut zum Ausbilden von physikalischem Plasma über größeren Flächen einer Kopfhaut eines Menschen zwecks Behandlung von Haarausfall bzw. Anregung von Haarwachstum geeignet ist.

### LÖSUNG

Die Aufgabe der Erfindung wird durch eine Vorrichtung mit den Merkmalen des unabhängigen Patentanspruchs 1 gelöst. Bevorzugte Ausführungsformen der erfindungsgemäßen Vorrichtung sind in den abhängigen Patentansprüchen definiert.

### BESCHREIBUNG DER ERFINDUNG

Bei einer erfindungsgemäßen Vorrichtung zum Ausbilden von physikalischem Plasma durch dielektrisch behinderte Entladungen gegenüber einer Oberfläche eines Objekts, die einen Hochspannungsanschluss und mehrere an den einen Hochspannungsanschluss kapazitiv angekoppelte, frei liegende Elektrodenoberflächen aufweisende und parallel zueinander zu distalen Enden hin langgestreckte Elektrodenkörper aufweist, sind an den distalen Enden Abstandhalter aus Dielektrikum angeordnet, die in Verlängerung der Elektrodenkörper 1,0 mm bis 5,0 mm über deren freiliegende Elektrodenoberflächen überstehen.

Bei der erfindungsgemäßen Vorrichtung sind die Elektrodenkörper durch die Abstandhalter aus Dielektrikum nicht mit einer dielektrischen Abschirmung versehen, die die durch Anlegen einer Hochspannung an die Elektrodenkörper hervorgerufenen elektrischen Entladungen dielektrisch behindert. Bei der erfindungsgemäßen Vorrichtung beruht die dielektrische Behinderung der Entladungen auf der kapazitiven Ankopplung der Elektrodenkörper an den Hochspannungsanschluss. Die Abstandhalter aus Dielektrikum stellen vielmehr einen definierten Abstand zwischen den frei liegenden Elektrodenoberflächen der Elektrodenkörper und der zu behandelnden Oberfläche des jeweiligen Objekts sicher, wenn die Abstandhalter der Oberfläche anliegen. Hieraus resultieren definierte Entladungsstrecken zwischen den frei liegenden Elektrodenoberflächen und der zu behandelnden Oberfläche des jeweiligen Objekts, über die das gewünschte physikalische Plasma unter definierten Bedingungen durch die dielektrisch behinderten elektrischen Entladungen erzeugt wird. Die wirksame Höhe der Abstandhalter der erfindungsgemäßen Vorrichtung von 1,0 bis 5,0 mm hält die zum Zünden von elektrischen Entladungen notwendige Hochspannung in Grenzen und entsprechend die durch diese Hochspannungen hervorgerufenen Reizungen der Oberfläche, beispielsweise einer menschlichen Kopfhaut. Zugleich wird aber sowohl ein elektrischer Kurzschluss zu der Oberfläche als auch ein lokal sehr eng begrenzter Entladungsbereich vermieden. Darüber hinaus schützen die Abstandhalter aus Dielektrikum die zu behandelnde Oberfläche vor einer mechanischen Beanspruchung durch die Elektrodenkörper. So besteht auch bei metallischen Elektrodenkörper geringen Durchmessers keine Gefahr einer kratzenden Beschädigung der Oberfläche.

Bei der erfindungsgemäßen Vorrichtung können die Abstandhalter auf die Elektrodenkörper aufgesetzt sein oder diese an ihren distalen Enden umfassen. Letzteres vergrößert die Kontaktfläche zwischen Abstandhaltern und den Elektrodenkörpern und verbessert damit die Dauerhaftigkeit der Verbindung zwischen den Abstandhaltern und den Elektrodenkörpern.

Die Abstandhalter können insbesondere kugel- oder tropfenförmig sein. Konkret können die Abstandhalter durch vorübergehendes Eintauchen der distalen Enden der Elektrodenkörper in eine Schmelze des jeweiligen Dielektrikums, nachfolgendes Ausformen der Abstandhalter aus der anhaftenden Schmelze durch die Oberflächenspannung der Schmelze und anschließendes Aushärten ausgebildet werden. Die Abstandhalter können jedoch nicht nur aus aufschmelzbarem Kunststoff, sondern auch aus anderen dielektrischen Materialien ausgebildet werden. In jedem Fall sorgen kugel- oder tropfenförmige Abstandhalter an den distalen Enden der Elektrodenkörper für einen hohen Verletzungsschutz der zu behandelnden Oberfläche durch die Elektrodenkörper.

Neben der Form wird der Verletzungsschutz durch die Abstandhalter auch von deren Oberflächenkrümmungsradien bestimmt. Vorzugsweise schließen die Abstandhalter mit Oberflächenkrümmungsradien ab, die so groß sind wie ein Durchmesser des jeweiligen Elektrodenkörpers im Bereich seiner frei liegenden Elektrodenoberfläche. Eine Obergrenze für die Oberflächenkrümmungsradien der Abstandhalter liegt etwa beim Dreifachen des Durchmessers des jeweiligen Elektrodenkörpers im Bereich seiner frei liegenden Elektrodenoberfläche. Bei einem kugelförmigen Abstandhalter weist dieser dann einen sechsmal so großen Durchmesser auf wie der Elektrodenkörper.

Im Bereich ihrer frei liegenden Elektrodenoberflächen können die Elektrodenkörper Durchmesser in einem typischen Bereich von 0,5 mm bis 2,5 mm aufweisen. Wenn die Elektrodenkörper beispielsweise aus Metall ausgebildet sind, bestimmt der Durchmesser neben dem Elastizitätsmodul des Metalls die Biegesteifigkeiten der Elektrodenkörper. Wie groß die Quersteifigkeit der Elektrodenkörper in Bezug auf Auslenkungen der Abstandhalter an ihrem freien Ende ist, hängt zusätzlich von der Länge der Elektrodenkörper ab. Diese Längen liegen in einem typischen Bereich von 5,0 mm bis 50 mm. Insgesamt ist es günstig, wenn die Elektrodenkörper elastisch sind und die Abstandhalter mit Biegesteifigkeiten von 600 Nmm² bis 4000 Nmm² abstützen. Damit ist die Lage der Abstandhalter noch ausreichend definiert, und sie können trotzdem beim Kontakt mit Hindernissen ausweichen. Durch diese Fähigkeit zum Ausweichen wird die Gefahr einer Beschädigung oder eines Ablösens der Abstandhalter von den Elektrodenkörpern deutlich reduziert.

Seitliche Abstände der Elektrodenkörper liegen in einem typischen Bereich von 2,0 mm bis 20 mm. Mit eben diesen seitlichen Abständen können die Abstandhalter in einem eindimensionalen Feld längs einer Geraden oder einer Linie mit stetigem Verlauf angeordnet sein. Eine solche stetige Linie weist keine Sprünge und vorzugsweise auch keine Knicke auf. In dieser Ausführungsform ähnelt die erfindungsgemäße Vorrichtung einem Kamm, dessen Zinken durch die Elektrodenkörper ausgebildet sind.

Bei einer bürstenartigen Ausführungsform der erfindungsgemäßen Vorrichtung sind die Abstandhalter in einem zweidimensionalen Feld längs einer Ebene oder einer Fläche mit stetigem Verlauf angeordnet. Auch eine solche stetige Fläche weist vorzugsweise nicht nur keine Sprünge, sondern auch keine Knicke auf. Die Fläche kann aber beispielsweise zweidimensional konkav gekrümmt sein, so dass sich die Abstandhalter an den distalen Enden der Elektrodenkörper großflächig an eine Kopfhaut anlegen können.

Vorzugsweise sind die Elektrodenkörper in einem Grundkörper der erfindungsgemäßen Vorrichtung abgestützt und im Bereich ihrer Abstützung einzeln kapazitiv an den einen Hochspannungsanschluss angekoppelt. Bevorzugt ist dabei eine individuelle Ankopplung der Elektrodenkörper an einen Hochspannungsbus, der an den einen Hochspannungsanschluss angeschlossen ist.

Der eine Hochspannungsanschluss kann seinerseits an einen Ausgang eines Hochspannungsgenerators der erfindungsgemäßen Vorrichtung angeschlossen sein, an dem der Hochspannungsgenerator eine gepulste Hochspannung gegenüber Erde ausgibt. Mit einer gepulsten Hochspannung, die aus Spannungspulsen gleichen oder wechselnden Vorzeichens gegenüber Erde zusammengesetzt sein kann, bei der es sich also um eine Gleich- oder eine Wechselspannung handeln kann, kann ein physikalisches Plasma mit geringerer elektrischer Leistung, also auch ohne signifikante Temperaturerhöhung ausgebildet werden, als beispielsweise mit einer sinusförmig modulierten Gleich- oder Wechselspannung. Ein kaltes Plasma ist jedoch wünschenswert, um thermische Effekte auf die zu behandelnde Oberfläche auszuschließen.

Konkret können die Pulse der gepulsten Hochspannung einen mittleren Pulsabstand aufweisen, der mindestens zehnmal, vorzugsweise mindestens hundertmal und noch mehr bevorzugt mindestens tausendmal so lang ist wie ihre mittlere Pulsdauer. Der mittlere Pulsabstand kann dabei bis zu hunderttausendmal oder sogar bis zu eine Millionen mal so lang sein wie die mittlere Pulsdauer.

Eine Erdung des Objekts, dessen Oberfläche mit der erfindungsgemäßen Vorrichtung behandelt wird, ist zumindest dann nicht notwendig, wenn der Hochspannungsgenerator Pulse wechselnden Vorzeichens, d. h. eine Wechselspannung ausgibt. Dann reichen kapazitive Eigenschaften des Körpers, dessen Oberfläche zu behandeln ist, für das Hervorrufen von elektrischen Entladungen zur Plasmaerzeugung aus.

Wie schon verschiedentlich angedeutet wurde, ist eine erfindungsgemäße Vorrichtung vorteilhaft als Haarausfalltherapiegerät nutzbar. Dabei dient sie zur Behandlung einer menschlichen Kopfhaut, um in dieser Gefäße zu weiten, eine Mikrozirkulation anzuregen und die Sauerstoffkonzentration zu erhöhen, so dass ein Haarwachstum gefördert wird.

Vorteilhafte Weiterbildungen der Erfindung ergeben sich aus den Patentansprüchen, der Beschreibung und den Zeichnungen.

Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen.

Hinsichtlich des Offenbarungsgehalts - nicht des Schutzbereichs - der ursprünglichen Anmeldungsunterlagen und des Patents gilt Folgendes: Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen, was aber nicht für die unabhängigen Patentansprüche des erteilten Patents gilt.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Hochspannungsbus die Rede ist, ist dies so zu verstehen, dass genau ein Hochspannungsbus, zwei Hochspannungsbusse oder mehr Hochspannungsbusse vorhanden sind. Die in den Patentansprüchen angeführten Merkmale können durch weitere Merkmale ergänzt werden oder die einzigen Merkmale sein, die das jeweilige Erzeugnis aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt in schematischer Darstellung eine erfindungsgemäße Vorrichtung beim Behandeln einer Kopfhaut.
- **Fig. 2**: ist eine perspektivische Ansicht einer beispielhaften Ausführungsform der erfindungsgemäßen Vorrichtung.
- **Fig. 3**: ist ein Schnitt durch die Vorrichtung gemäß Fig. 2 und
- **Fig. 4**: ist ein vergrößertes Detail der Vorrichtung gemäß Fig. 2, das in Fig. 3 mit einem Kreis markiert ist.

### FIGURENBESCHREIBUNG

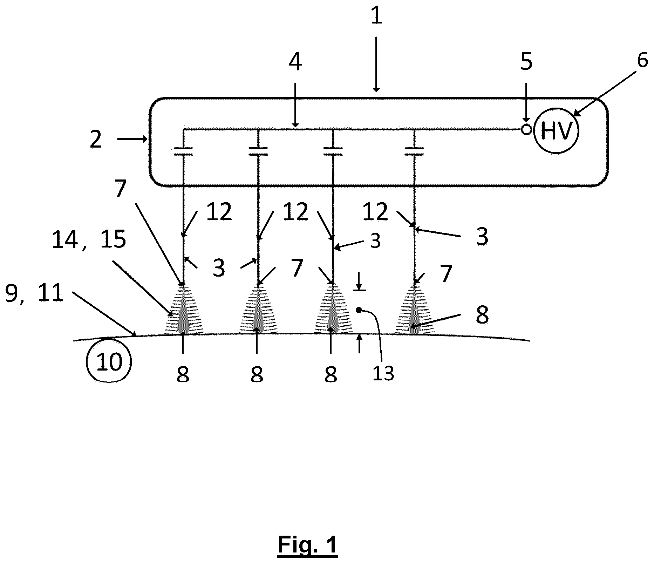

Die in **Fig. 1** dargestellte Vorrichtung 1 weist einen Grundkörper 2 auf. In dem Grundkörper 2 sind Elektrodenkörper 3 einzeln kapazitiv an einen Hochspannungsbus 4 angekoppelt, was durch Kondensator-Schaltzeichen angezeigt ist. Der Hochspannungsbus 4 ist an einen Hochspannungsanschluss 5 angeschlossen. Der Hochspannungsanschluss 5 wiederum ist an einen Ausgang eines Hochspannungsgenerators 6 angeschlossen. Hier ist der Hochspannungsgenerator 6 innerhalb des Grundkörpers 2 dargestellt; er kann aber auch von dem Grundkörper 2 getrennt sein. Der Hochspannungsgenerator 6 generiert an dem Hochspannungsanschluss 5 eine gepulste Hochspannung gegenüber Erde, deren Pulse wechselnde Vorzeichen der Hochspannung aufweisen. Die Elektrodenkörper 3 führen aus dem Grundkörper 2 heraus. Sie sind dabei parallel zueinander ausgerichtet und zu distalen Enden 7 hin langgestreckt. An den distalen Enden 7 sind Abstandhalter 8 angebracht. Die Abstandhalter 8 bestehen aus Dielektrikum. Über die Abstandhalter 8 liegen die Elektrodenkörper 3 an einer zu behandelnden Oberfläche 9 eines Objekts 10, hier einer Kopfhaut 11, an. Ein Überstand 13 der Abstandhalter 8 über die frei liegenden Elektrodenoberflächen 12 definiert den Abstand von frei liegenden Elektrodenoberflächen 12 zu der Oberfläche 9, über den hinweg sich beim Anlegen der gepulsten Hochspannung elektrische Entladungen 14 ausbilden. Diese elektrischen Entladungen 14 sind durch die kapazitive Ankopplung der Elektrodenkörper 3 an den Hochspannungsanschluss 5 dielektrisch behindert. Im Bereich der elektrischen Entladungen 14 bildet sich physikalisches Plasma 15. Das physikalische Plasma 15 bewirkt die gewünschte Behandlung der Oberfläche 9. Die hier tropfenförmig ausgebildeten Abstandhalter 8 bewirken auch eine Abstützung der Elektrodenkörper 3 an der Oberfläche 9, ohne die Gefahr von Verletzungen der Oberfläche 9. Der Überstand 13 beträgt zwischen 1,0 mm und 5,0 mm.

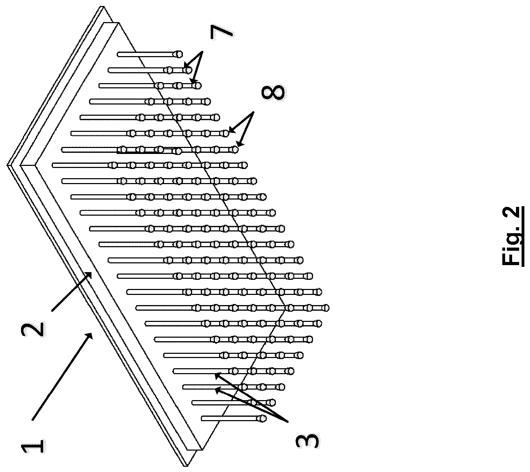

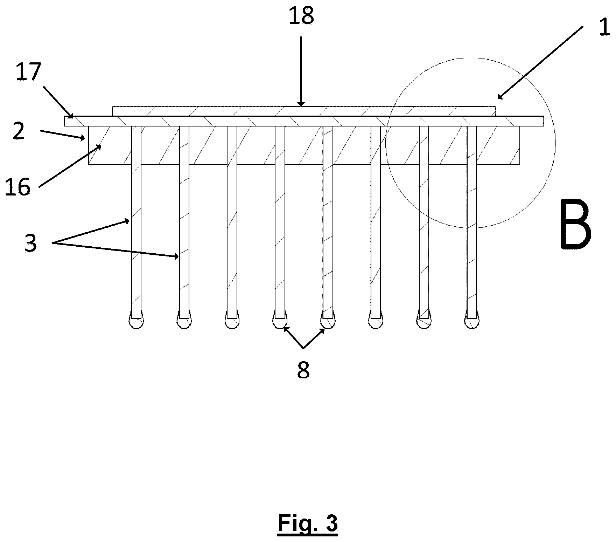

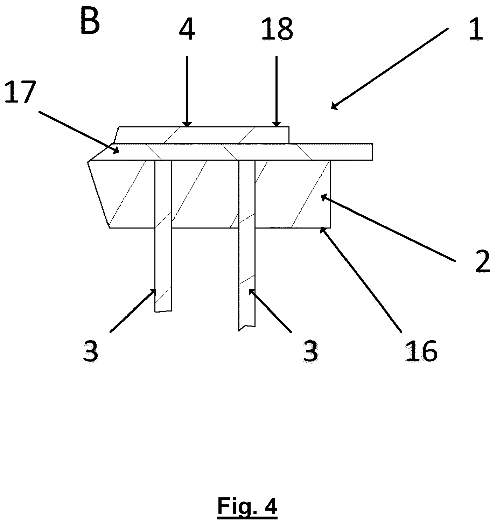

**Fig. 2** zeigt, dass die Elektrodenkörper 3 der erfindungsgemäßen Vorrichtung 1 nach Art der Borsten einer Bürste in einem zweidimensionalen Feld angeordnet sein können. Dabei liegen die distalen Enden 7 bzw. die hier kugelförmigen Abstandhalter 8 jeweils in einer parallel zu dem Grundkörper 2 verlaufenden Ebene. Der Schnitt gemäß **Fig. 3** und sein vergrößertes Detail gemäß **Fig. 4** zeigen, dass die einzelnen Elektrodenkörper 3 parallel zueinander in einer Platte 16 aus Dielektrikum verlagert sind und dabei bis zu einer durchgehenden Schicht 17, ebenfalls aus Dielektrikum reichen. Hinter der durchgehenden Schicht 17 ist eine weitere durchgehende Schicht 18 aus Metall angeordnet, die als der Hochspannungsbus 4 dient. Über die Schicht 17 hinweg sind die Elektrodenkörper 3 kapazitiv an den Hochspannungsbus 8 angekoppelt. Die Elektrodenkörper 3 weisen in dem gezeigten Ausführungsbeispiel einen Durchmesser von etwa 1 mm und einen seitlichen Abstand bzw. ein Rastermaß von etwa 5 mm auf. Die freien Längen der Elektrodenkörper 3 außerhalb des Grundkörpers 2 bis zu den Abstandhaltern 8 betragen etwa 20 bis 25 mm. Ein Durchmesser der hier kugelförmigen Abstandhalter 8 beträgt 2 bis 3 mm.

### BEZUGSZEICHENLISTE

- 1: Vorrichtung
- 2: Grundkörper
- 3: Elektrodenkörper
- 4: Hochspannungsbus
- 5: Hochspannungsanschluss
- 6: Hochspannungsgenerator
- 7: distales Ende
- 8: Abstandhalter
- 9: Oberfläche
- 10: Objekt
- 11: Kopfhaut
- 12: freiliegende Elektrodenoberfläche
- 13: Überstand
- 14: Entladung
- 15: Plasma
- 16: dielektrische Platte
- 17: dielektrische Schicht
- 18: metallische Schicht

## Patentansprüche

1. Vorrichtung (1) zum Ausbilden von physikalischem Plasma durch dielektrisch behinderte Entladungen gegenüber einer Oberfläche eines Objekts mit
- einem Hochspannungsanschluss (5),
- mehreren
- an den einen Hochspannungsanschluss (5) kapazitiv angekoppelten,
- frei liegende Elektrodenoberflächen (12) aufweisenden und
- parallel zueinander zu distalen Enden (7) hin langgestreckten Elektrodenkörpern (3) und
- Abstandhalter (8) aus Dielektrikum, die über die frei liegenden Elektrodenoberflächen (12) der Elektrodenkörper (3) überstehen,
**dadurch gekennzeichnet, dass** die Abstandhalter (8) aus Dielektrikum an den distalen Enden (7) der Elektrodenkörper (3) angebracht sind und in Verlängerung der Elektrodenkörper (3) 1,0 mm bis 5,0 mm über deren frei liegende Elektrodenoberflächen (12) überstehen.

2. Vorrichtung (1) nach Anspruch 1, **dadurch gekennzeichnet, dass** die Abstandhalter (8) auf die Elektrodenkörper (3) aufgesetzt sind und/oder diese umfassen.

3. Vorrichtung (1) nach Anspruch 1 oder 2, **dadurch gekennzeichnet, dass** die Abstandhalter (8) kugel- oder tropfenförmig sind.

4. Vorrichtung (1) nach einem der vorhergehenden Ansprüche, **dadurch gekennzeichnet, dass** die Abstandhalter (8) mit Oberflächenkrümmungsradien abschließen, die mindestens so groß sind wie ein Durchmesser des jeweiligen Elektrodenkörpers (3) im Bereich seiner frei liegenden Elektrodenoberfläche (12).

5. Vorrichtung (1) nach einem der vorhergehenden Ansprüche, **dadurch gekennzeichnet, dass** die Elektrodenkörper (3) im Bereich ihrer frei liegenden Elektrodenoberflächen (12) Durchmesser von 0,5 mm bis 2,5 mm aufweisen.

6. Vorrichtung (1) nach einem der vorhergehenden Ansprüche, **dadurch gekennzeichnet, dass** die Elektrodenkörper (3) freie Längen von 5,0 mm bis 50 mm aufweisen.

7. Vorrichtung (1) nach einem der vorhergehenden Ansprüche, **dadurch gekennzeichnet, dass** die Elektrodenkörper (3) elastisch sind und die Abstandhalter (8) mit Biegesteifigkeiten von 600 Nmm² bis 4000 Nmm² abstützen.

8. Vorrichtung (1) nach einem der vorhergehenden Ansprüche, **dadurch gekennzeichnet, dass** die Elektrodenkörper (3) in seitlichen Abständen von 2,0 mm bis 20 mm angeordnet sind.

9. Vorrichtung (1) nach einem der Ansprüche 1 bis 7, **dadurch gekennzeichnet, dass** die Abstandhalter (8) in einem eindimensionalen Feld längs einer Geraden oder einer Linie mit stetigem Verlauf angeordnet sind.

10. Vorrichtung (1) nach einem der Ansprüche 1 bis 7, **dadurch gekennzeichnet, dass** die Abstandhalter (8) in einem zweidimensionalen Feld längs einer Ebene oder einer Fläche mit stetigem Verlauf angeordnet sind.

11. Vorrichtung (1) nach einem der vorhergehenden Ansprüche, **dadurch gekennzeichnet, dass** die Elektrodenkörper (3) in einem Grundkörper (2) der Vorrichtung (1) abgestützt und in dem Grundkörper (2) kapazitiv an den einen Hochspannungsanschluss (5) angekoppelt sind.

12. Vorrichtung (1) nach einem der vorhergehenden Ansprüche, **dadurch gekennzeichnet, dass** der Hochspannungsanschluss (5) an einen Ausgang eines Hochspannungsgenerators (6) der Vorrichtung (1) angeschlossen ist, an dem der Hochspannungsgenerator (6) eine gepulste Hochspannung gegenüber Erde ausgibt.

13. Vorrichtung (1) nach Anspruch 12 **dadurch gekennzeichnet, dass** die gepulste Hochspannung eine Gleichspannung oder eine Wechselspannung ist.

14. Vorrichtung (1) nach Anspruch 13, **dadurch gekennzeichnet, dass** Pulse der gepulsten Hochspannung einen mittleren Pulsabstand aufweisen, der 10-mal bis 1.000.000-mal so lang ist wie ihre mittlere Pulsdauer.

15. Haarausfalltherapiegerät mit einer Vorrichtung (1) nach einem der vorhergehenden Ansprüche.

## Claims

1. Device (1) for generating physical plasma by means of dielectric barrier discharges with respect to a surface of an object, comprising
- a high voltage terminal (5),
- a plurality of electrode bodies (3)
- capacitively coupled to the one high voltage terminal (5),
- comprising exposed electrode surfaces (12) and
- elongated in parallel to one another towards distal ends (7), and
- spacers (8) made of dielectric which project beyond the exposed electrode surfaces (12) of the electrode bodies (3),
**characterized in that** the spacers (8) made of dielectric are attached to the distal ends (7) of the electrode bodies (3) and, in extension of the electrode bodies (3), project by 1.0 mm to 5.0 mm beyond the exposed electrode surfaces (12) of the electrode bodies (3).

2. Device (1) of claim 1, **characterized in that** the spacers (8) are arranged on top of the electrode bodies (3) and/or enclose them.

3. Device (1) of claim 1 or 2, **characterized in that** the spacers (8) are spherical or drop-shaped.

4. Device (1) of any of the preceding claims, **characterized in that** the spacers (8) end with surface curvature radiuses which are at least as long as a diameter of the respective electrode body (3) in the area of its exposed electrode surface (12).

5. Device (1) of any of the preceding claims, **characterized in that** the electrode bodies (3) in the area of their exposed electrode surfaces (12) have diameters from 0.5 mm to 2.5 mm.

6. Device (1) of any of the preceding claims, **characterized in that** the electrode bodies (3) have free lengths of 5.0 mm to 50 mm.

7. Device (1) of any of the preceding claims, **characterized in that** the electrode bodies (3) are elastic and support the spacers (8) at bending stiffnesses from 600 Nmm² to 4,000 Nmm².

8. Device (1) of any of the preceding claims, **characterized in that** the electrode bodies (3) are arranged at lateral distances from 2.0 mm to 20 mm.

9. Device (1) of any of the claims 1 to 7, **characterized in that** the spacers (8) are arranged in a one dimensional array along a straight line or a line with a steady course.

10. Device (1) of any of the claims 1 to 7, **characterized in that** the spacers (8) are arranged in a two dimensional array along a plane or an area with a steady course.

11. Device (1) of any of the preceding claims, **characterized in that** the electrode bodies (3) are mounted in a base body (2) of the device (1) and capacitively coupled to the one high voltage terminal (5) within the base body (2).

12. Device (1) of any of the preceding claims, **characterized in that** the high voltage terminal (5) is connected to an output of a high voltage generator (6) of the device (1) at which the high voltage generator (6) outputs a pulsed high voltage with respect to earth.

13. Device (1) of claim 12, **characterized in that** the pulsed high voltage is a direct voltage or an alternating voltage.

14. Device (1) of claim 13, **characterized in that** pulses of the pulsed high voltage have an average pulse spacing which is 10 times to 1,000,000 times as long as their average pulse duration.

15. Hair loss therapy apparatus comprising a device (1) of any of the preceding claims.

## Revendications

1. Dispositif (1) pour la formation d'un plasma physique par des décharges diélectriques incomplètes par rapport à une surface d'un objet avec
- un raccordement à haute tension (5),
- plusieurs éléments d'électrodes (3)
- couplés de manière capacitive au raccordement à haute tension (5),
- présentant des surfaces d'électrodes libres (12) et
- allongés parallèlement entre eux en direction d'extrémités distales (7)
et
- des entretoises (8) en diélectrique, qui dépassent des surfaces d'électrodes libres (12) des éléments d'électrodes (3),
**caractérisé en ce que** les entretoises (8) en diélectrique sont montées sur les extrémités distales (7) des éléments d'électrodes (3) et dépassent dans le prolongement des éléments d'électrodes (3) de 1,0 mm à 5,0 mm sur leurs surfaces d'électrodes libres (12).

2. Dispositif (1) selon la revendication 1, **caractérisé en ce que** les entretoises (8) sont posées sur les éléments d'électrodes (3) et/ou comprennent ceux-ci.

3. Dispositif (1) selon la revendication 1 ou 2, **caractérisé en ce que** les entretoises (8) présentent une forme sphérique ou une forme de goutte.

4. Dispositif (1) selon l'une des revendications précédentes, **caractérisé en ce que** les entretoises (8) se terminent par des rayons de courbures de surfaces qui sont au moins grands qu'un diamètre de l'élément d'électrode (3) correspondant au niveau de sa surface d'électrode libre (12).

5. Dispositif (1) selon l'une des revendications précédentes, **caractérisé en ce que** les éléments d'électrodes (3) présentent, au niveau de leurs surfaces d'électrodes libres (12), des diamètres de 0,5 mm à 2,5 mm.

6. Dispositif (1) selon l'une des revendications précédentes, **caractérisé en ce que** les éléments d'électrodes (3) présentent des longueurs libres de 5,0 mm à 50 mm.

7. Dispositif (1) selon l'une des revendications précédentes, **caractérisé en ce que** les éléments d'électrodes (3) sont élastiques et s'appuient sur les entretoises (8) avec des résistances à la flexion de 600 N/mm² à 4 000 N/mm².

8. Dispositif (1) selon l'une des revendications précédentes, **caractérisé en ce que** les éléments d'électrodes (3) sont disposés à des distances latérales de 2,0 mm à 20 mm.

9. Dispositif (1) selon l'une des revendications 1 à 7, **caractérisé en ce que** les entretoises (8) sont disposées, dans un champ monodimensionnel, le long d'une droite ou d'une ligne avec une extension latérale.

10. Dispositif (1) selon l'une des revendications 1 à 7, **caractérisé en ce que** les entretoises (8) sont disposées, dans un champ bidimensionnel, le long d'un plan ou d'une surface avec une extension latérale.

11. Dispositif (1) selon l'une des revendications précédentes, **caractérisé en ce que** les éléments d'électrodes (3) sont appuyés dans un corps de base (2) du dispositif (1) et sont couplés, dans le corps de base (2), de manière capacitive au raccordement à haute tension (5).

12. Dispositif (1) selon l'une des revendications précédentes, **caractérisé en ce que** le raccordement à haute tension (5) est raccordé à une sortie d'un générateur de haute tension (6) du dispositif (1), au niveau de laquelle le générateur de haute tension (6) émet une haute tension pulsée par rapport à la terre.

13. Dispositif (1) selon la revendication 12, **caractérisé en ce que** la haute tension pulsée est une tension continue ou une tension alternative.

14. Dispositif (1) selon la revendication 13, **caractérisé en ce que** des impulsions de la haute tension pulsée présentent une distance moyenne entre les impulsions qui est 10 fois à 1 000 000 de fois plus longue que leur durée moyenne d'impulsion.

15. Appareil de traitement anti-chute des cheveux avec un dispositif (1) selon l'une des revendications précédentes.
